(19) Europäisches Patentamt
European Patent Office
Office européen des brevets

(11) **EP 4 488 057 A1**

(12) **EUROPEAN PATENT APPLICATION**
published in accordance with Art. 153(4) EPC

(43) Date of publication:
**08.01.2025 Bulletin 2025/02**

(21) Application number: **23760023.4**

(22) Date of filing: **22.02.2023**

(51) International Patent Classification (IPC):
**B32B 5/26** (2006.01)   **A61F 13/00** (2024.01)
**B32B 5/02** (2006.01)   **D04H 1/4374** (2012.01)

(52) Cooperative Patent Classification (CPC):
**A61F 13/00; B32B 5/02; B32B 5/26; D04H 1/4374**

(86) International application number:
**PCT/JP2023/006412**

(87) International publication number:
**WO 2023/163024 (31.08.2023 Gazette 2023/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.02.2022 JP 2022029399**

(71) Applicant: **Kuraray Kuraflex Co., Ltd.
Okayama 702-8045 (JP)**

(72) Inventors:
• **MATSUSHITA, Kazuhiro
Okayama-shi, Okayama 702-8045 (JP)**

• **TOMOI, Masanori
Saijo-shi, Ehime 793-8585 (JP)**
• **ONO, Satoru
Okayama-shi, Okayama 702-8045 (JP)**
• **OCHIAI, Toru
Okayama-shi, Okayama 702-8045 (JP)**
• **OBATA, Soichi
Osaka-shi, Osaka 530-8611 (JP)**

(74) Representative: **Müller-Boré & Partner
Patentanwälte PartG mbB
Friedenheimer Brücke 21
80639 München (DE)**

(54) **FIBER SHEET**

(57) Provided is a fiber sheet having elasticity. The fiber sheet (10) includes at least: a first fiber layer (1) containing crimped fibers each having a coil shape, the first fiber layer (1) having repetitive elasticity in a longitudinal direction of the fiber sheet; and a second fiber layer (2) containing elastomeric fibers. The second fiber layer (2) is integrated with the first fiber layer (1) by fusion or adhesion in a specific thickness ratio therebetween.

Fig. 1

**Description**

CROSS REFERENCE TO THE RELATED APPLICATION

This application is based on and claims Convention priority to Japanese patent application No. 2022-29399, filed February 28, 2022, the entire disclosure of which is herein incorporated by reference as a part of this application.

FIELD OF THE INVENTION

The present invention relates to a fiber sheet that can achieve both improvement of repetitive elasticity and improvement of slip resistance or strength.

BACKGROUND OF THE INVENTION

Crimped fibers are fibers each of which is shrunk and curled into a coiled shape. Crimped fibers can be used for forming nonwoven fabrics, woven fabrics, knitted fabrics, and the like. For example, Patent Document 1 (JP Patent No. 6560683) discloses an elastic nonwoven fabric containing crimped fibers, as an elastic nonwoven fabric which has excellent repetition durability.

RELATED ART DOCUMENT

PATENT DOCUMENT

[Patent Document 1] JP Patent No. 6560683

SUMMARY OF THE INVENTION

Patent Document 1 describes that: it is possible to obtain an elastic nonwoven fabric that has excellent repetition durability, i.e., exhibits little deterioration in elasticity after repeated use; and that the elastic nonwoven fabric is useful as a bandage or the like. However, although the nonwoven fabric described in Patent Document 1 can exhibit excellent elasticity since the nonwoven fabric contains crimped fibers, the nonwoven fabric has the following drawback: when the nonwoven fabric is used, for example, as a bandage, the crimped fibers cannot produce slip resistance on the object (e.g., an arm or a leg) around which the nonwoven fabric is wrapped.

Therefore, one object of the present invention is to provide a fiber sheet that can achieve both elasticity and slip resistance.

As a result of extensive studies in order to achieve the above-mentioned object, the inventors of the present invention have found that under the following conditions that (i) in achieving slip resistance while taking advantage of repetitive elasticity exhibited by a fiber layer containing coil-like crimped fibers; (ii) a fiber layer is formed by fiberizing an elastomer resin; and furthermore, (iii) the fiber layer formed of the elastomer resin is integrated with a fiber layer containing coil-shaped crimped fibers by fusion or adhesion at a predetermined thickness ratio, the elastomer fibers can repeatedly expand and contract as well together with the coil-shaped crimped fibers, and as a result, the resulting fiber sheet has slip resistance while maintaining repetitive elasticity. The inventors of the present invention have further found that the combination of the fiber layer fiberized using the elastomer resin and the fiber layer containing the crimped fibers can improve strength of the fiber sheet while maintaining repetitive elasticity of the fiber sheet. Therefore, the inventors have completed the present invention.

Specifically, the present invention may include the following aspects.

[Aspect 1] A fiber sheet comprising at least:

a first fiber layer containing crimped fibers each having a coil shape, the first fiber layer having repetitive elasticity in a longitudinal direction of the fiber sheet; and
a second fiber layer containing an elastomer fiber, wherein
the second fiber layer is integrated with the first fiber layer by fusion or adhesion and
a thickness ratio of the second fiber layer to the first fiber layer, expressed as (second fiber layer) / (first fiber layer), is from 3/97 to 50/50 (preferably from 10/90 to 45/55, more preferably from 15/85 to 40/60, and further preferably from 15/85 to 30/70).

[Aspect 2] The fiber sheet according to aspect 1, wherein

the second fiber layer has a basis weight of 10 g/m$^2$ or higher (e.g., 10 to 250 g/m$^2$, preferably 30 to 250 g/m$^2$, more preferably 30 to 220 g/m$^2$, further preferably 30 to 200 g/m$^2$, even more preferably 45 to 180 g/m$^2$, and particularly preferably 50 to 150 g/m$^2$).

[Aspect 3] The fiber sheet according to aspect 1 or 2, wherein
the crimped fibers are derived from ester-based composite fibers.
[Aspect 4] The fiber sheet according to any one of aspects 1 to 3, wherein
the second fiber layer has a fused intersection between the elastomer fibers.
[Aspect 5] The fiber sheet according to any one of aspects 1 to 4, wherein
the elastomer fibers are styrene-based elastomer fibers.
[Aspect 6] The fiber sheet according to any one of aspects 1 to 5, wherein
a proportion of a bonding area between the second fiber layer and the first fiber layer is from 5 to 25% (preferably 8 to 20%) with respect to an area of an entire surface of the second fiber layer.
[Aspect 7] The fiber sheet according to any one of aspects 1 to 6, wherein
an interlayer peeling strength between the second fiber layer and the first fiber layer is 0.1 g/cm or higher (preferably 0.8 g/cm or higher and more preferably 1.0 g/cm or higher).
[Aspect 8] The fiber sheet according to any one of aspects 1 to 7, wherein
each of the first fiber layer and the second fiber layer is a nonwoven fabric.
[Aspect 9] The fiber sheet according to aspect 8, wherein
the first fiber layer is a hydroentangled nonwoven fabric, and the second fiber layer is a melt-blown nonwoven fabric.
[Aspect 10] The fiber sheet according to any one of aspects 1 to 9, comprising one or more first fiber layers and one or more second fiber layers,
wherein a second fiber layer is exposed on at least a part of at least one outermost surface of the fiber sheet.
[Aspect 11] The fiber sheet according to any one of aspects 1 to 10, wherein
the second fiber layer has an average fiber diameter of 1 to 40 μm (preferably 2.5 to 30 μm and more preferably 5 to 25 μm).
[Aspect 12] The fiber sheet according to any one of aspects 1 to 11, wherein
the first fiber layer has an average fiber diameter of 3.0 to 68.0 μm (preferably 7.0 to 30.5 μm, further preferably 9.5 to 21.5 μm, and particularly preferably 10.3 to 17 μm).
[Aspect 13] The fiber sheet according to any one of aspects 1 to 12, wherein
a proportion of the crimped fibers in the first fiber layer is 40% by mass or higher (preferably 55% by mass or higher, more preferably 65% by mass or higher, further preferably 80% by mass or higher, and particularly preferably 90% by mass or higher).
[Aspect 14] The fiber sheet according to any one of aspects 1 to 13, wherein
a basis weight ratio of the second fiber layer to the first fiber layer, expressed as (second fiber layer)/(first fiber layer), is 3/97 to 75/30 (preferably 3/97 to 70/30, more preferably 15/85 to 60/40, and further preferably 20/80 to 55/45).
[Aspect 15] The fiber sheet according to any one of aspects 1 to 14, wherein the fiber sheet is a bandage.

As used herein, the "longitudinal direction of the fiber sheet" may be one direction in a surface direction of the fiber sheet, and may typically be the longitudinal direction of the sheet. For example, the longitudinal direction may be the machine direction (MD) in a manufacturing process. In this case, a width direction may be a direction perpendicular to the longitudinal direction. For example, the width direction may be a direction (CD) orthogonally crossing the MD. In the case of "having repetitive elasticity in the longitudinal direction of the fiber sheet", the fiber sheet may have repetitive elasticity at least in the longitudinal direction, and may also have repetitive elasticity in another direction.

As used herein, singular forms used with the articles "a", "an", and "the" are intended to encompass plural forms including "at least one", unless the content clearly indicates otherwise. As used herein, the terms "and/or", "at least one", and "one or more" encompass any and all of combinations of the relevant items having been listed.

EFFECT OF THE INVENTION

According to the present invention, the fiber sheet can exhibit slip resistance while taking advantage of repetitive elasticity exhibited by the crimped fibers each having a coil shape.

BRIEF DESCRIPTION OF THE DRAWINGS

In any event, the present invention will be more clearly understood from the following description of preferred embodiments thereof, with reference to the accompanying drawings. However, the embodiments and the drawings are given only for the purpose of illustration and explanation, and are not to be taken as limiting the scope of the present invention in any way whatsoever, which scope is to be defined by the appended claims. In the accompanying drawings, like reference numerals are used to denote like parts throughout the several views, and:

Fig. 1 is a schematic diagram showing a schematic cross-sectional shape of a fiber sheet according to an embodiment of the present invention;

Fig. 2 is a schematic diagram showing a method for measuring fiber curvatures of crimped fibers in a fiber sheet according to the present invention;

Fig. 3 is a schematic perspective view showing a fiber sheet according to an embodiment of the present invention;

Fig. 4 is a schematic perspective view showing a fiber sheet according to an embodiment of the present invention;

Fig. 5 is a schematic perspective view showing a fiber sheet according to an embodiment of the present invention; and

Fig. 6 is a schematic diagram for explaining a method for measuring a bonding area between a first fiber layer and a second fiber layer.

DESCRIPTION OF EMBODIMENTS

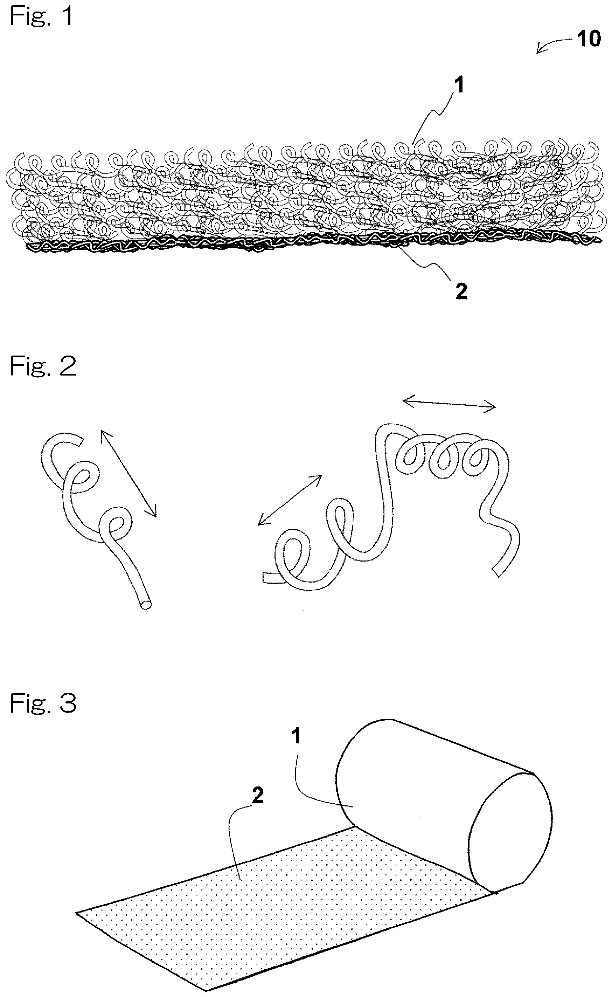

A fiber sheet according to the present invention includes at least a first fiber layer and a second fiber layer. Fig. 1 is a schematic diagram showing a schematic cross-sectional shape, in a thickness direction, of a fiber sheet according to an embodiment of the present invention. As shown in Fig. 1, for example, a fiber sheet 10 includes at least: a first fiber layer 1 containing crimped fibers each having a coil shape; and a second fiber layer 2 containing elastomer fibers. The first fiber layer and the second fiber layer 2 are integrated with each other by fusion or adhesion. The first fiber layer has repetitive elasticity in the longitudinal direction of the fiber sheet, due to the crimped fibers each having a coil shape.

[First Fiber Layer]

The first fiber layer includes crimped fibers each having a coil shape (hereinafter, sometimes referred to simply as crimped fibers). Due to the presence of the crimped fibers each having a coil shape, the first fiber layer can have repetitive elasticity.

The first fiber layer is not particularly limited as long as the first fiber layer is formed of fibers containing crimped fibers each having a coil shape. Examples of the first fiber layer can include woven fabrics, nonwoven fabrics, knits (knitted fabrics), and the like. Among these first fiber layers, a nonwoven fabric layer containing the crimped fibers each having a coil shape can be used. Such a first fiber layer can be obtained by, for example, performing entanglement treatment on a web containing potentially crimpable fibers and then by crimping the potentially crimpable fibers through heat treatment.

A web containing potentially crimpable fibers is not particularly limited as long as potentially crimpable fibers are included therein, and may be manufactured through various manufacturing methods. From the viewpoint of performing entanglement treatment, a web may preferably be formed through a dry process.

Specifically, potentially crimpable fibers and, as necessary, other fibers are mixed, and then the mixture is defibrated through carding with a carding machine, whereby a web is manufactured. Such a web may be any of: a parallel web in which fibers are arranged in the direction of process of the carding machine; a cross web in which parallel webs are cross-laid; a random web in which fibers are arranged randomly; or a semi-random web which is in the middle of a parallel web and a random web. A random web is preferable considering that the sheet can better conform to a surface in all directions when in use. Meanwhile, a semi-random web is preferable considering high productivity therefor.

A web containing potentially crimpable fibers may be subjected to entanglement treatment as necessary. In a case where a web is not subjected to entanglement treatment, crimping occurs (becomes prominent) during subsequent heat treatment which is carried out with less entanglement between the fibers. Consequently, a bulky, low-density sheet may be easily obtained.

In a case where a web is subjected to entanglement treatment, a method of mechanically entangling the fibers (e.g., needle punching) may be used, but hydroentanglement treatment is preferably performed. When water streams are utilized, entangled potentially crimpable fibers do not shrink individually when they are crimped by heat treatment, but rather shrink while maintaining the shape of the entire sheet, making it easier to obtain a uniform sheet.

Hydroentanglement treatment can be performed one or more times on one surface or both surfaces of a web which is placed on a movable support member (e.g., a belt conveyor). Water to be sprayed or jetted in hydroentanglement treatment (hereinafter, sometimes comprehensively referred to as "jetting") may be sprayed from one surface or both surfaces of a web.

From the viewpoint of improving elasticity after integration with the second fiber layer, a web may be at least loosely entangled, and the jetting pressure of water in hydroentanglement may be, for example, about 0.1 to 1.5 MPa, preferably about 0.3 to 1.2 MPa, and further preferably about 0.6 to 1.0 MPa.

Alternatively, ordinary entanglement treatment may be performed. In this case, water may be sprayed from one surface or both surfaces of a web at a jetting pressure of about 2 MPa or higher (e.g., 2 to 15 MPa), preferably about 3 to 12 MPa, and more preferably about 4 to 10 MPa (in particular, 5 to 8 MPa) in addition to or instead of the loose mild hydroentanglement.

The temperature of the water to be sprayed or jetted can be set as appropriate from the viewpoint of preventing crimping of potentially crimpable fibers at this stage. For example, the temperature may be about 5 to 50°C and preferably about 10 to 40°C (e.g., about 15 to 35°C (ambient temperature)).

Jetting of water is preferably performed through a method of jetting water onto a web from a plurality of holes having a regular spray range or spray pattern. The array or arrangement structure of the holes is not particularly limited, and may be, for example, a structure in which the holes are alternately arrayed in a netlike pattern or a lattice pattern (i.e., a staggered pattern). The holes typically have a same size, and the hole diameter may be, for example, about 1 to 10 mm and preferably about 1.5 to 5 mm from the viewpoint of, for example, adjusting the jetting pressure of water. The pitch between adjacent holes is also typically a same length, and the pitch may be, for example, about 1 to 5 mm and preferably about 1.5 to 3 mm.

Water may be jetted directly onto a web from a nozzle or may be jetted through a porous member (e.g., a perforated plate, a perforated plate drum). For example, a web may be placed on a support member (e.g., a belt conveyor), so that the web is passed between the porous member and the support member. A water-permeable support member is preferable in that water streams passing through the web can permeate the support member. Examples of the support member include a net coarser than roughly a 90-mesh net (e.g., a 10- to 80-mesh net). One belt conveyor may be used alone, or may be combined with another belt conveyor as necessary, such that the web is conveyed while being sandwiched between both belts.

A web in which fibers are entangled with each other is obtained through entanglement treatment. In potentially crimpable fibers contained in the web, crimping occurs (becomes prominent) during subsequent heat treatment, so that they become fibers having approximately coil-shaped (spiral or helical spring-shaped) three-dimensional crimps. In addition, in association with the shape change to an approximately coil shape, the fibers can also be entangled and restrained with each other.

Examples of the potentially crimpable fibers include composite fibers formed of a combination of resins having mutually different thermal shrinkage rates (or coefficients of thermal expansion), from the viewpoint of crimping the fibers through heat treatment. As resins for forming the composite fibers, two or more types of resins may be combined. A wide variety of fiber-forming resins can be used as resins for forming a composite resin, according to the difference in thermal shrinkage rates of resins.

Examples of the fiber-forming resins include: thermoplastic resins such as polyolefin-based resins (poly-$C_{2-4}$ olefin-based resins such as low-density, medium-density, or high-density polyethylene and polypropylene, and the like), acrylic-based resins (acrylonitrile-based resins each having an acrylonitrile unit, such as acrylonitrile-vinyl chloride copolymers, and the like), vinyl alcohol-based resins (thermoplastic polyvinyl alcohols, ethylene-vinyl alcohol copolymers, and the like), polyester-based resins (poly-$C_{2-4}$ alkylene arylate-based resins such as polyethylene terephthalate resins, polytrimethylene terephthalate resins, polybutylene terephthalate resins, polyethylene naphthalate resins, and the like), polyamide-based resins (aliphatic polyamide-based resins such as polyamide 6, polyamide 66, polyamide 11, polyamide 12, polyamide 610, and polyamide 612, semi-aromatic polyamide-based resins such as polyamide 9T and polyamide 6T, aromatic polyamide-based resins such as polyhexamethylene terephthalamide and polyhexamethylene isophthalamide, and the like), polyvinyl chloride-based resins (polyvinyl chloride, vinyl chloride-vinyl acetate copolymers, vinyl chloride-acrylonitrile copolymers, and the like), polyvinylidene chloride-based resins (vinylidene chloride-vinyl chloride copolymers, vinylidene chloride-vinyl acetate copolymers, and the like), styrene-based resins (heat-resistant polystyrene and the like), polycarbonate-based resins (bisphenol A polycarbonate and the like), phenoxy resins, polyphenylene sulfide resins, polyurethane-based resins, and cellulose-based resins (cellulose esters and the like); and modified resins of these thermoplastic resins; and the like. Among these resins, resins having a softening point or a melting point of 100°C or higher are preferable from the viewpoint of preventing excessive adhesion between the resins in heat treatment. In particular, polyester-based resins, polyamide-based resins, and vinyl alcohol-based resins are preferable from the viewpoint of excellent balance of heat resistance, fiber formability, and the like.

The transverse cross-sectional shape of a composite fiber (a cross-sectional shape taken in a direction perpendicular to the length direction of the fiber) is not limited to common solid cross-sectional shapes such as a round shape and an irregular shape [e.g., a flat shape, an elliptical shape, a polygonal shape, a 3- to 14-lobe shape, a T shape, an H shape, a V shape, a dog-bone shape (I shape), and the like]. The transverse cross-sectional shape may be a hollow cross-sectional shape or the like. However, usually, the transverse cross-sectional shape is a round shape.

The transverse cross-sectional structure of a composite fiber is not particularly limited as long as crimp properties are exhibited. The transverse cross-sectional structure may be a phase structure in which a plurality of resins exist as phase portions. Examples of the phase structure include structures of a core-sheath type, a sea-island type, a parallel type (or a side-by-side type or a multilayer laminated type), a radial type (or a radial laminated type), a hollow radial type, a block type, a random composite type, and the like. Among these transverse cross-sectional structures, a phase structure in which phase portions are asymmetrical with each other (e.g., a structure of an eccentric core-sheath type) and a phase structure in which phase portions are adjacent to each other (i.e., a structure of a so-called side-by-side type) are preferable because spontaneous crimping can be easily caused by heating.

Resins to be combined may have mutually different thermal shrinkage rates, and may be a combination of resins of the same type or a combination of resins of different types. For example, in a case where a composite fiber includes an outer phase and an inner phase (e.g., in the case of a core-sheath type structure, a sea-island type structure, or the like), the inner phase may be formed from a wet-heat adhesive resin (e.g., a vinyl alcohol-based polymer such as an ethylene-vinyl alcohol copolymer or a polyvinyl alcohol, or the like) or from a thermoplastic resin having a low melting point or softening point (e.g., polystyrene, low-density polyethylene, or the like), if there is a difference in thermal shrinkage from a resin located on the outer side and crimping can be achieved.

In a case where all of resin components are exposed on the outside (e.g., in the case of a parallel type structure), the resins are preferably brought into close contact with each other. In this case, the resin components are preferably composed of a combination of resins of the same type from the viewpoint of achieving close contact therebetween. As for a combination of resins of the same type, typically, a combination of a component (A) forming a homopolymer (essential component) and a component (B) forming a modified polymer (or copolymer) is used. That is, for example, a homopolymer as an essential component may be copolymerized and modified with a copolymerizable monomer, so that a difference in thermal shrinkage can be established between the component (A) and the component (B). The proportion of the copolymerizable monomer used for the modification to the total monomers is, for example, about 1 to 50 mol%, preferably about 2 to 40 mol%, and further preferably about 3 to 30 mol% (in particular, 5 to 20 mol%). The compounding ratio (mass ratio) of the component forming the homopolymer to the component forming the modified polymer can be selected according to the structure of the fibers. For example, the compounding ratio (mass ratio), expressed as homopolymer component (A) / modified polymer component (B), may be about 90/10 to 10/90, preferably about 70/30 to 30/70, and further preferably about 60/40 to 40/60.

From the viewpoint of ease of producing potentially crimpable composite fibers, composite fibers may be a combination of aromatic polyester-based resins. In particular, composite fibers may be a combination of a polyalkylene arylate-based resin (a) and a modified polyalkylene arylate-based resin (b). The polyalkylene arylate-based resin (a) may be a homopolymer of an aromatic dicarboxylic acid (a symmetrical aromatic dicarboxylic acid such as terephthalic acid or naphthalene-2,6-dicarboxylic acid, or the like) and an alkane diol component (a $C_{3-6}$ alkane diol such as ethylene glycol or butylene glycol, or the like). Specifically, a poly-$C_{2-4}$ alkylene terephthalate-based resin such as polyethylene terephthalate (PET) or polybutylene terephthalate (PBT), or the like, is used. Usually, PET used for common PET fibers having an intrinsic viscosity of about 0.6 to 0.7 is used.

Meanwhile, as the modified polyalkylene arylate-based resin (b), it is possible to use a copolymerization component that decreases the melting point or the softening point, and/or the crystallinity of the polyalkylene arylate-based resin (A) which is an essential component: e.g., a dicarboxylic acid component such as an asymmetrical aromatic dicarboxylic acid, an alicyclic dicarboxylic acid, or an aliphatic dicarboxylic acid, or an alkane diol component and/or an ether-bond-containing diol component having a longer chain length than the alkane diol of the polyalkylene arylate-based resin (a). These types of copolymerization components may be used singly, or two or more of these types of copolymerization components may be used in combination. Among these components, commonly used dicarboxylic acid components may be an asymmetrical aromatic carboxylic acid (isophthalic acid, phthalic acid, 5-sodium sulfoisophthalate, or the like), an aliphatic dicarboxylic acid (a $C_{6-12}$ aliphatic dicarboxylic acid such as adipic acid), or the like, and commonly used diol components may be an alkane diol (a $C_{3-6}$ alkane diol such as 1,3-propanediol, 1,4-butanediol, 1,6-hexanediol, or neopentyl glycol, or the like), a polyoxyalkylene glycol (a polyoxy-$C_{2-4}$ alkylene glycol such as diethylene glycol, triethylene glycol, polyethylene glycol, or polytetramethylene glycol, or the like), or the like. Among these commonly-used components, an asymmetrical aromatic dicarboxylic acid such as isophthalic acid, a polyoxy-$C_{2-4}$ alkylene glycol such as diethylene glycol, or the like is preferable. Furthermore, the modified polyalkylene arylate-based resin (b) may be an elastomer containing a $C_{2-4}$ alkylene arylate (ethylene terephthalate, butylene terephthalate, or the like) as a hard segment; and (poly) oxyalkylene glycol or the like as a soft segment.

With the modified polyalkylene arylate-based resin (b) containing dicarboxylic acid components, the proportion of the amount of a dicarboxylic acid component(s) (e.g., isophthalic acid or the like) for decreasing the melting point or the softening point to the total amount of the dicarboxylic acid components may be, for example, about 1 to 50 mol%, preferably about 5 to 50 mol%, and further preferably about 15 to 40 mol%. With the modified polyalkylene arylate-based resin (b) containing diol components, the proportion of the amount of a diol component(s) (e.g., diethylene glycol or the like) for decreasing the melting point or the softening point to the total amount of the diol components may be, for example, 30 mol% or lower and preferably 10 mol% or lower (e.g., about 0.1 to 10 mol%). When the proportion of the copolymerization component is excessively low, crimps do not sufficiently develop, resulting in lower form stability and elasticity of the nonwoven fabric after crimp development. Meanwhile, when the proportion of the copolymerization component is excessively high, high crimping performance can be obtained, but it becomes difficult to perform stable spinning.

The modified polyalkylene arylate-based resin (b) may be branched, if necessary, by using in combination a polyvalent carboxylic acid component such as trimellitic acid or pyromellitic acid, a polyol component such as glycerin, trimethylolpropane, trimethylolethane, or pentaerythritol, or the like.

The fiber length of crimped fibers can be selected from, for example, a range of about 10 to 100 mm. The fiber length may be preferably about 20 to 80 mm and further preferably about 25 to 75 mm (in particular, 40 to 60 mm). In a case where the fiber length is larger than the lower limit, a fiber web can be easily formed. In addition to this advantage, when crimping occurs, crimped fibers are sufficiently entangled with each other, whereby strength and elasticity of the nonwoven fabric can be improved. Meanwhile, in a case where the fiber length is smaller than the upper limit, a fiber web having a uniform basis weight can be easily formed, and fibers are moderately entangled with each other at the time of web formation, so that elasticity can be easily exhibited. Furthermore, when the fiber length falls within the above range, a part of fibers crimped at a surface of an elastic nonwoven fabric is moderately exposed on the surface of the nonwoven fabric, whereby self-adhesiveness of the elastic nonwoven fabric can be improved.

Crimped fibers may be mechanically crimped before heating. The number of crimps resulting from the mechanical crimping may be, for example, about 0 to 30 crimps/25 mm, preferably about 1 to 25 crimps/25 mm, and more preferably about 5 to 20 crimps/25 mm.

A web may be formed of potentially crimpable fibers alone or may be formed from a combination of potentially crimpable fibers and non-crimped fibers. Examples of the non-crimped fibers include: cellulose-based fibers [e.g., natural fibers (cotton, wool, silk, hemp, or the like), semi-synthetic fibers (acetate fibers such as triacetate fibers, and the like), regenerated fibers (rayon, polynosic, cupra, or lyocell (e.g., "Tencel", which is a registered trademark name, and the like), and the like)]; polyolefin-based fibers such as polypropylene fibers and polyethylene fibers; polyester fibers; polyamide fibers; acrylic fibers; and the like. These types of fibers may be used singly, or two or more of these types of fibers may be used in combination. These fibers can be selected as appropriate according to required characteristics of the fiber sheet. Among these fibers, cellulose-based fibers are preferable from the viewpoint of improving hygroscopicity and mixability with crimped fibers.

From the viewpoint of obtaining a favorable mixability with crimped fibers, the fiber length of non-crimped fibers can be selected from, for example, a range of about 10 to 100 mm. The fiber length may be preferably about 20 to 80 mm and further preferably about 25 to 75 mm (in particular, 30 to 60 mm).

In heat treatment, potentially crimpable fibers (composite fibers) in a web are crimped. Heating may be performed by dry-heat treatment. However, heating may preferably be performed by using high-temperature steam. In the treatment with high-temperature steam, a web conveyed by a belt conveyor is exposed to a high-temperature or heated steam (high-pressure steam) flow, which causes composite fibers (potentially crimpable fibers) to develop crimps. The heat treatment causes crimping of the composite fibers (potentially crimpable fibers). As a result, the shapes of the composite fibers can be changed to coil shapes, and three-dimensional entanglement of the fibers can be achieved by utilizing the coil shapes.

It is preferable that a web treated with low-pressure water is subjected to high-temperature steam treatment on a belt conveyor, and the web is shrunk upon receiving the high-temperature steam treatment. Therefore, the web being supplied is, desirably overfed according to the intended size of the nonwoven fabric, immediately before being exposed to high-temperature steam. The overfeeding proportion is about 110 to 300% and preferably about 120 to 250% of the intended length of the nonwoven fabric.

The belt conveyor to be used is not particularly limited as long as, basically, the belt conveyor can convey a web to be processed without disturbing the form of the web. An endless conveyor may be suitably used. The belt conveyor may be a general single belt conveyor. Alternatively, another belt conveyor may be combined therewith as necessary such that a web is conveyed while being sandwiched between both belts. By conveying the web in such a way, the form of the web which is being conveyed can be inhibited from being unintentionally deformed, due to an external force such as high-temperature steam or vibrations of the conveyor when the web is treated. In the case of using a pair of belts, the distance between the belts may be selected as appropriate according to the desired basis weight and density of the web, and may be, for example, about 1 to 10 mm, preferably about 1 to 8 mm, and more preferably about 1 to 5 mm.

A commonly-used steam injection device is used for supplying steam to a web. The steam injection device is preferably a device capable of spraying the steam approximately uniformly over the entire width of the web at a desired pressure and amount.

In the case of combining two belt conveyors, the steam injection device is mounted in one of the conveyors, and steam is supplied to the web through a water-permeable conveyor belt or a conveyor net placed on the conveyor. A suction box may be mounted to the other conveyor as necessary.

Excess steam having passed through a web may be suctioned and discharged by the suction box. However, in order to sufficiently adhere steam to a web and to more efficiently cause fiber crimping due to the heat of steam, the web needs to be kept in as free a state as possible, and thus steam is preferably supplied without being suctioned and discharged by the suction box.

Furthermore, in order to perform steam treatment on the front and back sides of the web at once, the conveyor located on the opposite side to the conveyor on which the aforementioned steam injection device is mounted may be provided with another steam injection device which may be mounted at a downstream portion of the conveyor relative to a position at which the aforementioned steam injection device is mounted. If it is desired to perform steam treatment on the front and back sides of a nonwoven fabric in a case where there is no steam injection device at the downstream portion, as another option, a web which have been treated once may be turned upside down and be passed through the treatment device again.

The endless belt used in a conveyor is not particularly limited as long as it does not interfere conveyance of a web and high-temperature steam treatment. However, if a net is used as an endless belt, a net coarser than roughly 90-mesh net (e.g., a roughly 10- to 50-mesh net) is preferable. A finer net having a smaller mesh size has lower air permeability and makes it difficult for steam to pass through the net. From the viewpoint of, for example, heat resistance against steam treatment, the material of the belt may be preferably a metal or a heat-resistant resin such as a thermally-protected polyester-based resin, a polyphenylene sulfide-based resin, a polyarylate-based resin (wholly aromatic polyester-based resin), an aromatic polyamide-based resin, or the like.

High-temperature steam jetted from the steam injection device is in the form of an air flow, and therefore, unlike in hydroentanglement treatment or needle-punch treatment, the high-temperature steam enters the inside of a web as a treatment-target object without significantly moving the fibers in the web. It is considered that, due to the effect of steam flow entering a web, the steam flow efficiently covers the surface of each fiber existing in the web, permitting uniform thermal crimping. In addition, heat can be transferred sufficiently to the inside of the fibers when compared to dry-heat treatment, whereby generally uniform crimping can be achieved on the surface and in the thickness direction.

The nozzle for jetting high-temperature steam may be a plate or a die having predetermined orifices arranged one after another in the width direction, and the nozzle may be disposed such that the orifices are arranged in the width direction of a web to be supplied. One or more orifice rows may be required, and a plurality of orifice rows may be arranged in parallel. A plurality of nozzle dies each having one row of orifices may be disposed in parallel.

When using a nozzle plate in the form of a plate with orifices, the thickness of the plate may be about 0.5 to 1.0 mm. There are no particular limitations on the diameter and the pitch of the orifices as long as desired crimp development and fiber entanglement associated therewith can be efficiently achieved. However, from the viewpoint of securing steam jetting force, the diameter of an orifice may be about 0.05 to 2 mm, preferably about 0.1 to 1 mm, and further preferably about 0.2 to 0.5 mm. From the viewpoint of improving permeability of steam into a web, the pitch between the orifices may be usually about 0.5 to 3 mm, preferably about 1 to 2.5 mm, and further preferably about 1 to 1.5 mm.

The pressure at which high-temperature steam is jetted is not particularly limited as long as intended fiber crimping and moderate fiber entanglement associated therewith can be achieved, and may be set according to the material and the form of fibers to be used. However, the pressure may be, for example, about 0.1 to 2 MPa, preferably about 0.2 to 1.5 MPa, and further preferably about 0.3 to 1 MPa.

The temperature of high-temperature steam may be, for example, about 70 to 150°C, preferably about 80 to 120°C, and further preferably about 90 to 110°C. The speed of treatment with high-temperature steam may be, for example, about 200 m/min or lower, preferably about 0.1 to 100 m/min, and further preferably about 1 to 50 m/min.

As moisture may remain in a nonwoven fabric after crimping of composite fibers in the web is developed, the nonwoven fabric may be dried as necessary.

A crimped fiber having a coil shape is a fiber having at least a portion that is approximately coil-shaped (spiral shaped or coil spring shaped) to form one or more turns of coil crimps (hereinafter, sometimes referred to as coil portion), for example, as shown in Fig. 2. Fig. 2 shows crimped fibers having coil portions, which are taken out of the first fiber layer. In Fig. 2, the fibers have, at the coil portions thereof, axial directions indicated by the double-headed arrows. An example of the axial directions may be as follows. That is, where a single fiber has different coil portions, the coil portions may have mutually different axial directions.

The above-mentioned composite fibers are potentially crimpable fibers. By performing heat treatment thereto, crimps develop (or become prominent), and the composite fibers become fibers having approximately coil-shaped (spiral or helical spring-shaped) three-dimensional crimps. The number of crimps in a crimped fiber after heating may be, for example, 30 crimps/25 mm or more (e.g., 30 to 200 crimps/25 mm), preferably about 35 to 150 crimps/25 mm, more preferably about 40 to 120 crimps/25 mm, about 45 to 120 crimps/25 mm (in particular, 50 to 100 crimps/25 mm).

Since the first fiber layer includes crimped fibers each having a coil shape, the first fiber layer can have a structure in which the crimped fibers are entangled with each other at the coil portions thereof so as to be restrained or engaged. In particular, the first fiber layer may be such that, in the course of shrinkage of fibers into coil shapes in a manufacturing process for the fiber layer, the fibers are entangled with each other and the crimped fibers are restrained by the entangled coil portions.

The first fiber layer may be formed of crimped fibers alone or may be formed from a combination of crimped fibers and non-crimped fibers as discussed above. The proportion of the crimped fibers in the first fiber layer may be, for example, 40% by mass or higher, preferably 55% by mass or higher, more preferably 65% by mass or higher, further preferably 80% by mass or higher, and particularly preferably 90% by mass or higher. The balance between strength and elasticity or flexibility of the first fiber layer can be adjusted by, for example, mixing the non-crimped fibers according to the purpose.

From the viewpoint of achieving both the repetitive elasticity and breaking strength of the first fiber layer, the average fiber diameter of fibers forming the first fiber layer can be selected from, for example, a range of about 3.0 to 68.0 $\mu$m and may be preferably about 7.0 to 30.5 $\mu$m, further preferably about 9.5 to 21.5 $\mu$m, and particularly preferably about 10.3 to 17 $\mu$m. The average fiber diameter is a value measured according to a method explained in EXAMPLES described later.

In the first fiber layer, from the viewpoint of increasing elasticity in a surface direction, it is preferable that crimped fibers (in particular, axial directions of coil portions of crimped fibers) are oriented in the surface direction of the fiber layer more so than in the thickness direction of the fiber layer. Also, crimped fibers that are adjacent to or intersect with each other are preferably entangled with each other at the crimped coil portions thereof. When a higher proportion of the crimped fibers are oriented in the surface direction of the fiber layer, repetitive elasticity in the surface direction of the fiber sheet can be improved. Also, from the viewpoint of increasing cushioning properties, crimped fibers are preferably entangled with each other also in the thickness direction (or an oblique direction) of the nonwoven fabric, but to a smaller extent than in the surface direction of the nonwoven fabric.

Crimped fibers (in particular, axial directions of coil portions of the crimped fibers) are preferably oriented mainly in one direction of the surface direction. In this case, elasticity in said direction is improved. Thus, when a tension is applied in said direction, the entangled crimped coil portions can be elongated through elastic deformation, and, when a tension is further applied, the entangled crimped coil portions can be elongated through plastic deformation. Meanwhile, when a tension is no longer applied, the entangled crimped coil portions can be swiftly restored to the original shape. Here, the term "mainly" means an amount (by mass, if necessary) that is more than half the total amount of the crimped fibers.

In the first fiber layer, crimped fibers (axial directions of coil portions of the crimped fibers) are preferably oriented substantially parallel to a fiber layer surface (sheet surface).

Consequently, the first fiber layer can be preferably elongated more in the surface direction than in the thickness direction due to entangled crimped coil portions. In addition, in the first fiber layer, crimped fibers are preferably oriented in a sheet longitudinal direction of the surface direction. Therefore, when a tension is applied in the sheet longitudinal direction, the entangled crimped coil portions are elongated but, at the same time, attempt to be restored to the original coil shapes, whereby high elasticity can be exhibited in the longitudinal direction of the surface direction. Furthermore, since crimped fibers are entangled with each other to a small extent in the thickness direction of the fiber layer, cushioning properties and flexibility can be exhibited in the thickness direction, whereby the first fiber layer can have comfortable touch and texture.

The first fiber layer may contain a commonly-used additive. Examples of the commonly-used additive include stabilizers (e.g., heat stabilizers such as copper compounds, ultraviolet absorbers, light stabilizers, antioxidants, and the like), antibacterial agents, deodorants, fragrances, colorants (e.g., dyes/pigments and the like), fillers, antistatic agents, flame retardants, plasticizers, lubricants, crystallization rate retarders, and the like. The additives can be used alone or in combination of two or more. The additive may be carried on the surface of the fibers or may be contained in the fibers.

[Second Fiber Layer]

The second fiber layer is not particularly limited as long as it contains elastomer fibers, and the second fiber layer can be a woven fabric, a nonwoven fabric, a knit (knitted fabric), and the like. The second fiber layer may be directly joined to the first fiber layer. Alternatively, the second fiber layer may be joined to the first fiber layer via an adhesive.

The second fiber layer contains elastomer fibers. The proportion of the elastomer fibers in the second fiber layer may be, for example, 40% by mass or higher, preferably 55% by mass or higher, more preferably 65% by mass or higher, further preferably 80% by mass or higher, and particularly preferably 90% by mass or higher. Examples of non-elastomer fibers include the fibers presented as examples of the non-crimped fibers, and the like.

From the viewpoint of effectively utilizing repetitive elasticity of crimped fibers existing in the first fiber layer, the second fiber layer preferably has a nonwoven fabric structure with intersections resulting from fusion between the elastomer fibers. In a case where a nonwoven fabric layer is used as the second fiber layer, examples of the nonwoven fabric layer include melt-blown nonwoven fabrics, electrospun nonwoven fabrics, spun-bonded nonwoven fabrics, and the like. Among these nonwoven fabrics, melt-blown nonwoven fabrics are preferable from the viewpoint of handleability.

A melt-blown nonwoven fabric can be obtained by a melt-blowing method in which an elastomer resin composition is melted; the molten material extruded from a spinneret is subjected to hot air jets to be thinned into fibers, and the resulting fibers are formed into a nonwoven fabric by utilizing self-fusing properties of the fibers.

In this case, the second fiber layer may be produced by directly blowing molten elastomer fiber onto the first fiber layer as a support (direct blow method).

Examples of the elastomer fibers can include thermoplastic elastomer fibers such as styrene-based elastomer fibers, urethane-based elastomer fibers, olefin-based elastomer fibers, ester-based elastomer fibers, polyvinyl chloride-based elastomer fibers, and amide-based elastomer fibers. From the viewpoint of excellent elasticity, examples of preferable elastomer fibers include styrene-based elastomer fibers and urethane-based elastomer fibers. In particular, from the viewpoint of excellent slip resistance on the skin, styrene-based elastomer fibers are preferable.

Styrene-based elastomer fibers may be formed from a resin composition containing: a styrene-based elastomer resin containing a block copolymer composed of a polymer block (A) containing at least two aromatic vinyl compounds as main components and a polymer block (B) containing at least one conjugated diene compound as a main component and/or a hydrogenated product of the block copolymer; as well as a softener for the styrene-based elastomer resin as necessary.

A particularly preferable styrene-based elastomer resin to be used for the styrene-based elastomer resin composition is composed of polystyrene as a hard segment and polybutadiene, polyisoprene, hydrogenated polybutadiene, polyethylene, polypropylene, or the like as a soft segment. Examples of the particularly preferable styrene-based elastomer resin include SBS (styrenebutadiene-styrene block copolymer), SIS (styrene-isoprene-styrene block copolymer), SEBS (styrene-ethylene-butadiene-styrene block copolymer), SEPS (styrene-ethylene-propylene-styrene block copolymer), and the like. These types of copolymers may be used alone, or in combination of two or more.

A urethane-based elastomer forming urethane-based elastomer fibers may be formed from a resin composition containing a urethane-based elastomer resin composed of a hard segment formed from a low-molecular-weight glycol and a diisocyanate and a soft segment formed from a high-molecular-weight diol and a diisocyanate; and a softener for the urethane-based elastomer resin as necessary.

Examples of the low-molecular-weight glycol include: $C_{1-10}$ diols such as ethylene glycol, 1,4-butanediol, and 1,6-hexanediol; and the like. Examples of the high-molecular-weight diol include poly(1,4-butylene adipate), poly(1,6-hexane adipate), polycaprolactone, polyethylene glycol, polypropylene glycol, polyoxytetramethylene glycol, and the like. Examples of the diisocyanate include tolylene diisocyanate, 4,4-diphenylmethane diisocyanate, hexamethylene diisocyanate, isophorone diisocyanate, and the like.

Amide-based elastomer fibers may be formed from a resin composition containing an amide-based elastomer resin composed of a polyamide (e.g., a $C_{6-22}$ aliphatic polyamide and preferably a $C_{9-20}$ aliphatic polyamide) as a hard segment and an amorphous polyether or polyester with a low glass transition temperature as a soft segment; and a softener for the amide-based elastomer resin as necessary.

Ester-based elastomer fibers may be formed from a resin composition containing an ester-based elastomer resin composed of a saturated polyester as a hard segment and an aliphatic polyether or an aliphatic polyester as a soft segment; and a softener for the ester-based elastomer resin as necessary.

Polyvinyl chloride-based elastomer fibers may be formed from a resin composition containing a polyvinyl chloride-based elastomer resin composed of crystalline polyvinyl chloride as a hard segment and amorphous polyvinyl chloride or acrylonitrile as a soft segment; and a softener for the polyvinyl chloride-based elastomer resin as necessary.

Olefin-based elastomer fibers may be formed from a resin composition containing an olefin-based elastomer resin composed of polyethylene or polypropylene as a hard segment and SEBS or an ethylene-propylene copolymer as a soft segment; and a softener for the olefin-based elastomer resin as necessary.

A resin composition forming fibers of the second fiber layer may be a polymer blend that contains an elastomer resin in combination with another polymer.

The second fiber layer may contain a known or conventional additive applied to the inside or outside of the fibers as necessary. Examples of the additive include flame retardants, antibacterial agents, light stabilizers, ultraviolet absorbers, antioxidants, release agents, lubricants, colorants, antistatic agents, and the like.

From the viewpoint of improving followability to crimped fibers having a coil shape, fibers forming the second fiber layer may have an average fiber diameter of, for example, about 1 to 40 $\mu$m, preferably about 2.5 to 30 $\mu$m, and more preferably about 5 to 25 $\mu$m. The average fiber diameter is a value measured according to a method explained in EXAMPLES described later.

From the viewpoint of increasing followability to the first fiber layer, the second fiber layer may have a basis weight of, expressed as a basis weight ratio (second fiber layer)/(first fiber layer), for example, 3/97 to 75/30, preferably 3/97 to 70/30, more preferably 15/85 to 60/40, and further preferably 20/80 to 55/45.

From the viewpoint of reinforcing repetitive elasticity, the second fiber layer may have a basis weight of, for example, 10 g/m$^2$ or higher (e.g., 10 to 250 g/m$^2$), preferably 30 to 250 g/m$^2$, more preferably 30 to 220 g/m$^2$, further preferably 30 to 200 g/m$^2$, even more preferably 45 to 180 g/m$^2$, and particularly preferably 50 to 150 g/m$^2$.

(Thickness Ratio between First Fiber Layer and Second Fiber Layer)

The thickness ratio of the second fiber layer to the first fiber layer, expressed as (second fiber layer)/(first fiber layer), is 3/97 to 50/50. Such a thickness ratio makes it possible to exhibit slip resistance while maintaining repetitive elasticity of the first fiber layer.

In addition, by controlling the thickness ratio of the second fiber layer to the first fiber layer breaking strength of the fiber sheet can be improved by the second fiber layer while maintaining repetitive elasticity of the first fiber layer.

The thickness ratio, expressed as (second fiber layer)/(first fiber layer), may be preferably 10/90 to 45/55, more preferably 15/85 to 40/60, and further preferably 15/85 to 30/70.

Specifically, the thickness of the second fiber layer may be set as appropriate according to the thickness of the first fiber layer and may be, for example, 0.05 to 0.80 mm, preferably 0.10 to 0.70 mm, and further preferably 0.15 to 0.60 mm.

(Integration of First Fiber Layer and Second Fiber Layer)

The first fiber layer and the second fiber layer may be integrated with each other by directly discharging a resin melt for forming the second fiber layer onto the first fiber layer as a support, whereby the second fiber layer and the first fiber layer are integrated with each other. Alternatively, the first fiber layer and the second fiber layer may be separately produced and then be integrated with each other in a subsequent step.

In the case of performing the integration in a subsequent step, the first fiber layer and the second fiber layer may be adhered to each other by a known pressure-sensitive adhesive or the like. Alternatively, the first fiber layer and the second fiber layer may be fused to each other through heat treatment such as heat roller treatment or heat embossing treatment. Fusion by means of heating is preferable from the viewpoint of utilizing heat fusibility of the elastomer fibers forming the second fiber layer. Hereinafter, fusion and adhesion are sometimes collectively referred to as joining.

The first fiber layer and the second fiber layer may have approximately same areas, and may form joined portions of approximately same areas. Alternatively, out of the first fiber layer and the second fiber layer, one fiber layer may have a larger area than the other fiber layer. In this case, the one fiber layer having a larger area may be exposed and form joined portions with the other fiber layer.

Regarding the joined portions, the entireties of the joined portions may be continuously joined together, or the joined portions may be intermittently joined together in a desired pattern (e.g., a dotted pattern) or the like. In the case of intermittent joining, an adhesive may be applied onto at least one of the fiber layers to perform the joining in a desired pattern, or both nonwoven fabrics may be joined together by thermocompression bonding, such as heat embossing. In heat embossing, convex portions are preferably pushed from the second fiber layer toward the first fiber layer. In this case, the fiber sheet may have a structure in which a portion of the elastomer fibers in the second fiber layer enter as a melt, the inside of the first fiber layer.

Also, the second fiber layer may have substantially a same area as the first fiber layer and may be provided over the entire surface of the first fiber layer, or may cover a portion of the first fiber layer to leave an exposed portion. For example, Fig. 3 to Fig. 5 schematically show examples of joining patterns between the first fiber layer and the second fiber layer in the fiber sheet. These fiber sheets are each shown as being rolled up in the longitudinal direction.

For example, in the fiber sheet as shown in Fig. 3, the second fiber layer 2 is joined to the entirety of one surface of the first fiber layer 1. In Fig. 3, the first fiber layer 1 and the second fiber layer 2 are intermittently joined together in the overlapping portions thereof, and the dots on the second fiber layer 2 indicate joined points (e.g., thermocompression bonded points formed through embossing treatment). Each of the dots simply indicates a schematic position thereof and does not indicate the size or shape of an actual joined point, and the same applies below.

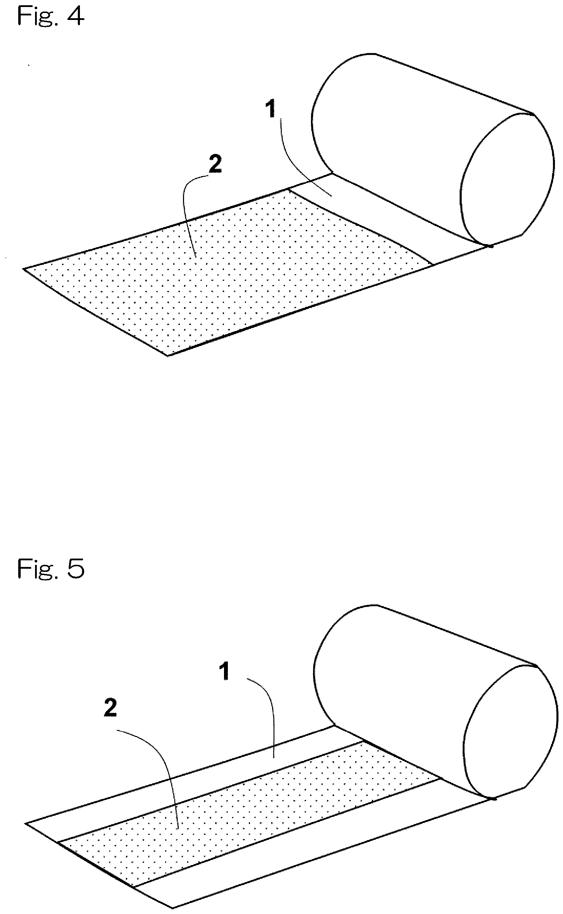

Alternatively, as shown in Fig. 4, in the fiber sheet, the second fiber layer 2 may be disposed on one end portion in the longitudinal direction of one surface of the first fiber layer. For example, at least the second fiber layer may be provided so that, when such a fiber sheet is used to wrap an object, the second fiber layer 2 as a wrapping start part is brought into contact with the object at a wrapping start point, and the first fiber layer 1 may be exposed so that the first fiber layer 1 as a wrapping end part is brought into contact with the opposite surface of an already-wrapped portion of the first fiber layer at a wrapping end point. In this way, it is possible to finish wrapping by utilizing self-adhesiveness between the first fiber layers.

Alternatively, as shown in Fig. 5, in the fiber sheet, the second fiber layer may be disposed at a center portion of the first fiber layer while the first fiber layer is exposed at both ends in the width direction on one surface of the first fiber layer. In each of Figs. 4 and 5 as well, the first fiber layer 1 and the second fiber layer 2 are intermittently joined together in the overlapping portions thereof, and the dots on the second fiber layer 2 indicate joined points.

Coil portions of crimped fibers forming the first fiber layer have excellent entanglement properties by itself, and therefore, can be easily entangled with other crimped coil portions by contact at a certain pressure. As a result, the first fiber layers can exhibit self-adhesiveness. Therefore, in the case of the fiber sheet as shown in Fig. 4 or Fig. 5, when the fiber sheet is used to wrap an object, the second fiber layer is brought into contact with the object at the wrapping start, so that slip resistance of the second fiber layer can be used to efficiently start wrapping the object, and self-adhesiveness between an exposed portion of the first fiber layer and an already-wrapped portion of the first fiber layer of the fiber sheet can be utilized at the wrapping end to complete the wrapping of the object with the fiber sheet.

From the viewpoint of improving elasticity of the second fiber layer, the first fiber layer and the second fiber layer may be preferably intermittently joined together. More preferably, joined portions may be regularly scattered.

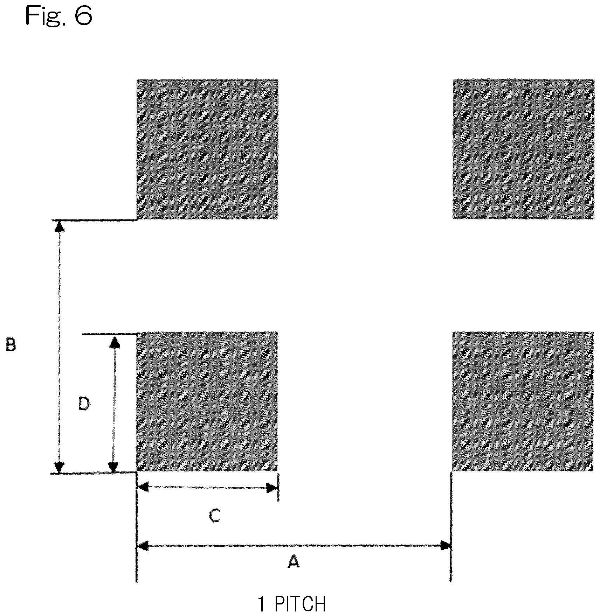

For example, in a case where the first fiber layer and the second fiber layer are intermittently joined together, the fiber sheet may have a joining area in a proportion of 5 to 25% and preferably 8 to 20% of overlapping portions of the first fiber layer and the second fiber layer. The proportion of a joining area means the proportion of the area of a joined point, at which the first fiber layer and the second fiber layer are joined together by fusion or adhesion, to a predetermined area which is extracted from a whole pattern of intermittently joined points and which can be determined to represent the whole. For example, in the case of a pattern of regularly arranged joined points as in Fig. 6, the proportion of a joining area indicates the proportion of the area of a joined point to the total area of the joined point and a non-joined point at one pattern portion in the pattern of regularly arranged joined points. In Fig. 6, rectangular joined points are regularly arranged, and thus the proportion of a joining area can be calculated by using the following formula, as the proportion of the area (C×D) of the joined point to the total area (A×B) of the joined point and the non-joined point.

$$\text{Proportion of joining area} = (C{\times}D)/(A{\times}B){\times}100 \ (\%)$$

Meanwhile, the interlayer peeling strength between the second fiber layer and the first fiber layer in at least one direction (e.g., MD: a product flow direction or a machine advancing direction) may be, for example, 0.1 g/cm or higher, preferably 0.8 g/cm or higher, and more preferably 1.0 g/cm or higher. A higher interlayer peeling strength is more preferable, and the upper limit thereof may be, for example, about 5 g/cm. The interlayer peeling strength is a value measured according to a method explained in EXAMPLES described later.

A case where the fiber sheet has a two-layer structure composed of the first fiber layer and the second fiber layer has been described above. However, in the present invention, the fiber sheet may include one or more first fiber layers and one or more second fiber layers. In this case, it is preferable that a second fiber layer is at least partially exposed on at least one outermost surface of the fiber sheet from the viewpoint of slip resistance.

The basis weight of the whole fiber sheet may be, for example, 50 to 500 g/m$^2$, preferably 55 to 400 g/m$^2$, more preferably 60 to 350 g/m$^2$, and particularly preferably 60 to 300 g/m$^2$.

On one hand, the fiber sheet may have a breaking strength (tensile breaking stress) of 5 N/50 mm or higher in at least one direction (e.g., MD) of the surface direction in an overlapping portion between the first fiber layer and the second fiber layer. The fiber sheet may have a breaking strength preferably 10 N/50 mm or higher, and more preferably 15 N/50 mm or higher. Although the upper limit of the breaking strength is not particularly limited, and the upper limit may be, for example, 100 N/50 mm or lower.

On the other hand, the breaking strength of the fiber sheet can also be increased by the second fiber layer in a direction other than at least one direction of the above-mentioned surface directions, for example, in a direction (CD) perpendicular to the machine direction (MD) in the manufacturing process, in an overlapping portion between the first fiber layer and the second fiber layer. The breaking strength may be, for example, 1 N/50 mm or higher, preferably 2 N/50 mm or higher, more preferably 4 N/50 mm or higher, and further preferably 5 N/ mm or larger. Although the upper limit of the breaking strength is not particularly limited, the upper limit may be, for example, 100 N/50 mm or lower as described above.

The fiber sheet may have an elongation rate of at least 50% in at least one direction (e.g., MD) of the surface direction in an overlapping portion between the first fiber layer and the second fiber layer.

Also, the fiber sheet may preferably have a recovery rate of 70% or more (100% or less) after 10 cycles of 50% elongation in at least one direction (e.g., MD) in the surface direction (i.e., a recovery rate after 10 cycles of 50% elongation). The recovery rate after 10 cycles of 50% elongation may be more preferably 80% or higher, and further preferably 85% or higher. The recovery rate after 10 cycles of 50% elongation is a value measured according to a method explained in EXAMPLES described later.

The fiber sheet may have an elongation rate of at least 100% in a direction other than at least one direction, for example, in a direction (CD) perpendicular to the machine direction (MD) in the manufacturing process, in an overlapping portion between the first fiber layer and the second fiber layer.

Also, the fiber sheet may preferably have a recovery rate of 45% or higher (100% or lower) after 10 cycles of 100% elongation in the direction (e.g., CD) other than the at least one direction in the surface direction (i.e., a recovery rate after 10 cycles of 100% elongation). The recovery rate after 10 cycles of 100% elongation may be more preferably 60% or higher, further preferably 70% or higher, even more preferably 75% or higher, and particularly preferably 85% or higher. The recovery rate after 10 cycles of 100% elongation is a value measured according to a method explained in EXAMPLES described later.

The second fiber layer in the fiber sheet may have a surface friction coefficient, as an index of slip resistance, of, for example, 4.0 or more, preferably 5.0 or larger, and more preferably 6.0 or more. Also, the upper limit value of the surface friction coefficient is not particularly limited and may be, for example, about 8.0. The surface friction coefficient of the second fiber layer is a value measured according to a method explained in EXAMPLES described later.

The external shape of the fiber sheet according to the present invention can be selected according to the application, and may be a long rectangular sheet (bandage-shaped sheet) or a small or large sheet having a predetermined shape. Such a fiber sheet can be used for various applications: for instance, household items such as anti-slips for rugs; sporting goods such as training mats, sports grips, underwraps (bands as a base for taping), and pressure tapes; clothing items such as shoe insoles s, anti-slips for socks, and trouser grips; medical, cosmetic, and hygiene materials such as bandages and upper arm anti-sagging tapes; and the like.

Especially, the fiber sheet is particularly useful as a medical, cosmetic, or hygienic fiber sheet for single-handed application from the viewpoint of having slip resistance and repetitive elasticity. Examples of the single-handed application include an instance of applying such a sheet to a site which a person is unable to apply it by themselves using both hands and has to apply it using one hand. The application site may be, for example, the shoulder, upper arm, forearm, elbow, wrist, hand, or the like. For example, where the fiber sheet is used as bandages or upper arm anti-sagging tapes, or the like, the second fiber layer of the fiber sheet exhibits slip resistance on the object (e.g., the upper arm) at the beginning of wrapping and allows the fiber sheet to be easily wrapped, even in a case where only one hand can be used.

EXAMPLES

Hereinafter, the present invention will be further specifically described by presenting Examples, but the present invention is not limited to these Examples. Each of physical property values in the following Examples and Comparative Examples was measured by the following methods.

[Average Fiber Diameter]

The surface of each layer was observed by using a scanning electron microscope. The diameters of 100 fibers randomly selected from electron micrographs were measured, and the number-average fiber diameter was determined, which was taken as the average fiber diameter of the fibers.

[Number of Crimps Resulting from Mechanical Crimping (Number Of Crimps)]

Measurement was performed according to "Test methods for man-made staple fibres" (8.12.1) of JIS L 1015.

[Basis Weight]

Measurement was performed according to "Test methods for general nonwovens" of JIS L 1913.
For a fiber sheet in which the first fiber layer and the second fiber layer are integrated with each other, the fiber layers may be separated into individual layers for the moment by peeling off, and then the basis weight of each of the layers may be measured and be used as the basis weight of each layer.

[Thickness]

By using a razor ("Feather Razor S with a single edge" manufactured by FEATHER Safety Razor Co., Ltd.), each of the fiber sheets was, at ten arbitrarily-selected locations thereof, cut in a direction that was parallel to the thickness direction of the fiber sheet and that was perpendicular to the machine (flow) direction (MD). At the ten locations, the respective cross-sections were observed in digital microscope photographs. For a fiber sheets in which the first fiber layer and the second fiber layer were integrated with each other, respective thicknesses of the layers were measured through the observation of their cross sections in the integrated state by using a microscope (KEYENCE DEGITAL MICROSCOPE VHX-900), and the average value of the thicknesses was used as the thickness of the layer.

[Breaking Strength and Breaking Elongation]

Measurement was performed according to "Test methods for general nonwovens" of JIS L 1913. An AG-IS manufactured by Shimadzu Corporation was used as a constant speed extension type tensile tester. Five test pieces each having a width of 50 mm and a length of 200 mm were prepared, each test piece having a longitudinal direction corresponding to the flow direction (MD) at the time of manufacturing of fiber sheets. After the gripping interval was set to 10 cm, an end portion of each test piece was fixed by a gripping portion of the tester and was pulled at a speed of 200 mm/min until the test piece was broken. The average value (n=5) of maximum loads at break was used as a breaking strength. Breaking strength and breaking elongation were measured in each of the flow direction (MD) and the width direction (CD) of the nonwoven fabric.

[Interlayer peeling Strength]

Three test pieces each having a width of 50 mm and a length of 200 mm were prepared, each test piece having a longitudinal direction corresponding to the flow direction (MD) at the time of manufacturing of fiber sheets. An AG-IS manufactured by Shimadzu Corporation was used as the constant speed extension type tensile tester. Kraft tape was applied to the end of each prepared test piece, the layers of the test piece were peeled off from each other to the length between the chucks, and the layers were fixed by gripping portions and pulled at a speed of 200 mm/min up to a stroke of 120 mm. The average of strengths other than the strengths at stroke portions of an initial stroke of 20 mm and a final stroke of 50 mm was measured at N=3, and the average value was used as a peeling strength.

[Recovery rate after 10 cycles of 50% elongation]

Measurement was performed according to "Test methods for general nonwovens" of JIS L 1913. Five test pieces each having a width of 50 mm and a length of 200 mm were prepared, each test piece having a longitudinal direction corresponding to the flow direction (MD) at the time of manufacturing of fiber sheets. An AG-IS manufactured by Shimadzu Corporation was used as a constant speed extension type tensile tester. Each of the test pieces was pulled in the longitudinal direction at a speed of 200 mm/min. The load was removed immediately after the elongation rate reached 50%. After restoration, the next operation (elongation) was performed without any standby time. After the operations were repeated 10 times, a tensile test was performed, and a recovery rate after 10 cycles of 50% elongation was determined by using the following formula:

$$\text{Recovery rate after 10 cycles of 50\% elongation (\%)} = 100\text{-}X.$$

In the formula, X represents a residual strain (%) after 10 cycles of 50% elongation.

[Recovery rate after 10 cycles of 100% elongation]

Measurement was performed according to "Test methods for general nonwovens" of JIS L 1913. Five test pieces each having a width of 50 mm and a length of 200 mm were prepared, each test piece having a longitudinal direction corresponding to a direction (CD) orthogonally crossing the flow direction (MD) at the time of manufacturing of fiber sheets. An AG-IS manufactured by Shimadzu Corporation was used as the constant speed extension type tensile tester. Each of the test pieces was pulled in the longitudinal direction at a speed of 200 mm/min. The load was removed immediately after the elongation rate reached 100%. After restoration, the next operation (elongation) was performed without any standby time. After the operations were repeated 10 times, a tensile test was performed, and a recovery rate after 10 cycles of 100% elongation was determined by using the following formula:

$$\text{Recovery rate after 10 cycles of 100\% elongation (\%)} = 100\text{-}Y.$$

In the formula, Y represents a residual strain (%) after 10 cycles of 100% elongation.

[Surface Friction Coefficient]

Measurement was performed with reference to "Plastics - Film and sheeting - Determination of the coefficients of friction" of JIS K 7125 (1999). Five test pieces each extending over 15 cm in the MD and each extending over 25 cm in the CD were prepared, each test piece having a longitudinal direction corresponding to the flow direction (MD) at the time of manufacturing of fiber sheets.

A digital force gauge (ZTS) and a measurement stand (MH2-500N) manufactured by IMADA Co., Ltd. were used as measurement devices. The measurement was performed using the following method. The CD was set to coincide with the longitudinal direction of the measurement stand. The digital force gauge (ZTS) was set on the measurement stand. A weight (1000 g) was placed on each measurement sample, and measurement was performed at a movement speed of 200 mm/sec. The average of values from 5 seconds to 15 seconds after the start of the measurement was used as a friction resistance.

The following raw materials were prepared as raw materials for use in the Examples and the Comparative Examples.

[Potentially Crimpable Fibers]

Side-by-side type composite staple fibers composed of a polyethylene terephthalate resin [component (A)] having an intrinsic viscosity of 0.65 and a modified polyethylene terephthalate resin [component (B)] obtained by copolymerizing 20 mol% of isophthalic acid and 5 mol% of diethylene glycol (average fiber diameter: 13.1 $\mu$m (1.7 dtex), fiber length: 51 mm, number of crimps resulting from mechanical crimping: 12 crimps/25 mm, number of crimps after heat treatment at 130°C for 1 minute: 62 crimps/25 mm)

[Rayon Fibers]

Regenerated cellulose fibers "HOPE" manufactured by Omikenshi Co., Ltd. (average fiber diameter: 12.6 μm, fiber length: 40 mm)

[Polyester Fibers]

Polyethylene terephthalate (PET) fibers manufactured by TORAY INDUSTRIES, INC. (average fiber diameter: 12.8 μm, fiber length: 51 mm)

[Example 1]

(Preparation of First Fiber Layer)

A carded web having a basis weight of 32.1 g/m$^2$ was obtained using 100% by mass of the potentially crimpable fibers by a carding method. The carded web was moved on a conveyor net so as to pass between the conveyor net and a perforated plate drum having (circular) holes with a diameter φ of 2 mm and a pitch of 2 mm in a staggered pattern, and water flow was sprayed at 0.8 MPa from the inside of the perforated plate drum toward the web and the conveyor net. Consequently, the fibers were wetted to the extent that no substantial entanglement of the fibers occurred and the fibers moved slightly.

Then, this carded web was transferred to a belt conveyor equipped with a 30-mesh, 500 mm wide resinous endless belt. In this step, the web was overfed by about 200% so as not to inhibit shrinkage during the subsequent steam treatment step. This belt conveyor was equipped with a same belt located above the aforementioned belt, and they were rotated in the same direction at the same speed. The belt conveyor used allowed the gap between the belts to be arbitrarily adjusted.

Then, the web was introduced to a steam injection device provided to the belt conveyor, and steam treatment was performed such that steam was injected at 0.4 MPa from the steam injection device in a direction perpendicular to the web. Consequently, the potentially crimpable fibers developed coil-shaped crimps, and the fibers were entangled to obtain a nonwoven fabric. The steam injection device had nozzles installed in one of the conveyors so as to spray steam toward a web through the conveyor belt, and a suction device was installed in the other conveyor. However, this suction was not put into operation. The device had steam injection nozzles with a hole diameter of 0.3 mm and the nozzles were arranged in one row at a pitch of 2 mm along the width direction of the conveyor. The treatment speed was set to 10 m/min, and the distance between each nozzle and the conveyor belt on the suction side was set to 10 mm. The obtained crimped-fiber nonwoven fabric had a basis weight of 90.0 g/m$^2$.

When the surface and cross section in the thickness direction of the obtained nonwoven fabric were observed with an electron microscope (100-fold), it was found that each of the fibers was oriented substantially parallel to the surface direction of the nonwoven fabric.

(Preparation of Second Fiber Layer)

Using 100 parts by mass of a styrene-based elastomer resin ("EARNESTON (registered trademark)" CJ101 manufactured by KURARAY PLASTICS CO., Ltd.), melt-blown spinning was performed using general melt-blowing manufacturing equipment, with 400 spinning holes (arranged in a single row) at a spinning temperature of 300°C, an air pressure of 4 MPa, a hole diameter φ of 0.3 mm and a single hole discharge rate of 0.4 g/hole/min. With the distance from the nozzle to the belt conveyor set to 200 mm, the fibers were collected at a speed of 4 m/min by using a rotating net conveyor as a support, whereby an elastomer-fiber nonwoven fabric (basis weight: 100 g/m$^2$) was produced.

(Compositing)

The obtained crimped-fiber nonwoven fabric and the elastomer-fiber nonwoven fabric were set on an unrolling machine and were supplied to undergo a thermocompression bonding step while being unrolled at an unrolling speed of 3.5 m/min. In the thermocompression bonding step, compositing by thermocompression bonding was performed using upper and lower rolls, with the elastomer-fiber nonwoven fabric being located on the upper side and with the crimped-fiber nonwoven fabric being located on the lower side. The upper roll had embossing projections (in a deformed quadrangular pattern having a pressure-bonding area of 14.87%), and the lower roll was flat. The compositing was performed at a pressure of 2.3 kg/cm$^2$ (50 kg/cm), with the upper roll having a set temperature of 125°C and with the lower roll having a set temperature of 0°C. The composite fiber sheet was wound up by a winding machine.

[Example 2]

A fiber sheet was obtained in the same manner as in Example 1, except that the following urethane-based elastomer-fiber nonwoven fabric was used as a second fiber layer. The second fiber layer was a nonwoven fabric of urethane elastomer fibers with a basis weight of 70 g/m$^2$, which was obtained by using 100 parts by mass of a polyurethane resin (PANDEX T-1190KS manufactured by DIC Covestro Polymer Ltd.), by performing melt-blown spinning using general melt-blowing manufacturing equipment, with 400 spinning holes (arranged in a single row) at a spinning temperature of 243°C, an air pressure of 0.4 MPa, a hole diameter φ of 0.3 mm and a single hole discharge rate of 0.2 g/hole/min, and by collecting the fibers at a speed of 8 m/min with a rotating net conveyor as a support. This urethane-based elastomer-fiber nonwoven fabric was used as a second fiber layer.

[Example 3]

A fiber sheet was obtained in the same manner as in Example 1, except that the crimpable fibers used for the first fiber layer with the fineness of 1.2 dtex were manufactured to obtain a crimped-fiber nonwoven fabric having a basis weight of 90 g/m$^2$.

[Example 4]

A fiber sheet was obtained in the same manner as in Example 1, except that a crimped-fiber nonwoven fabric used as the first fiber layer was produced by mixing 60% by mass of the potentially crimpable fibers and 40% by mass of the rayon fibers as the potentially non-crimpable fibers, to obtain the crimped-fiber nonwoven fabric having a basis weight of 100 g/m$^2$.

[Example 5]

A fiber sheet was obtained in the same manner as in Example 1, except that the speed of the net conveyor at the time of production of the second fiber layer was set to 10 m/min, the elastomer-fiber nonwoven fabric used as the second fiber layer had a basis weight of 40 g/m$^2$.

[Example 6]

A fiber sheet was obtained in the same manner as in Example 1, except that: unlike the crimpable fibers used for the first fiber layer, the fineness of the potentially crimpable fibers was set to 0.9 dtex; and a nonwoven fabric was produced as follows.

(Preparation of First Fiber Layer)

A carded web having a basis weight of 90 g/m$^2$ was obtained using 100% by mass of the potentially crimpable fibers by a carding method. The carded web was placed on a punched drum support having an open area ratio of 25% and a hole diameter of 0.3 mm, and then was continuously transferred in the longitudinal direction at a speed of 50 m/min while high-pressure water streams were injected from the above so as to perform the entanglement treatment. In this entanglement treatment, two nozzles were used (the distance between the adjacent nozzles: 20 cm), in which orifices each having a hole diameter of 0.10 mm were provided at an interval of 0.6 mm along the width direction of the web. High-pressure water streams were injected from the nozzle in the first row at a water pressure of 3.0 MPa; and high-pressure water streams were injected from the nozzle in the second row at a water pressure of 4.0 MPa. The obtained nonwoven fabric sheet was transferred onto a belt conveyor equipped with a 30-mesh, 500 mm wide resin endless belt. In this step, the web was overfed by about 200% so as not to inhibit shrinkage during the subsequent steam treatment step. This belt conveyor was equipped with a same belt located above the aforementioned belt, and they were rotated in the same direction at the same speed. The belt conveyor used allowed the gap between the belts to be arbitrarily adjusted.

Then, the carded web was introduced to a steam injection device provided to the belt conveyor, and steam treatment was performed such that steam was injected at 0.4 MPa from the steam injection device in a direction perpendicular to the carded web. Consequently, the potentially crimpable fibers developed the coil-shaped crimps to obtain a nonwoven fabric.

The steam injection device had nozzles installed in one of the conveyors so as to spray steam toward the web through the conveyor belt, and a suction device was installed on the other conveyor. However, this suction was not put into operation. The device had steam injection nozzles with a hole diameter of 0.3 mm and the nozzles were arranged in one row at a pitch of 2 mm along the width direction of the conveyor. The treatment speed was set to 10 m/min, and the distance between each nozzle and the conveyor belt on the suction side was set to 10 mm. The obtained crimped-fiber nonwoven fabric had a basis weight of 90.0 g/m$^2$.

[Example 7]

A fiber sheet was obtained in the same manner as in Example 6, except that the crimped-fiber nonwoven fabric used as the first fiber layer was produced by mixing 50% by mass of the potentially crimpable fibers and 50% by mass of the rayon fibers as potentially non-crimpable fibers, to obtain the obtained crimped-fiber nonwoven fabric having a basis weight of 100 g/m$^2$.

[Example 8]

A fiber sheet was obtained in the same manner as in Example 1, except that the speed of the net conveyor at the time of production of the second fiber layer was set to 2 m/min, the elastomer-fiber nonwoven fabric used as the second fiber layer had a basis weight of 200 g/m$^2$.

[Example 9]

A fiber sheet was obtained in the same manner as in Example 1, except that: the speed of the net conveyor at the time of production of the second fiber layer was set to 2 m/min; the elastomer-fiber nonwoven fabric used as the second fiber layer had a basis weight of 200 g/m$^2$; and the distance from each nozzle to the belt conveyor was set to 400 mm.

[Example 10]

To obtain a crimped-fiber nonwoven fabric as the first fiber layer, 70% by mass of the potentially crimpable fibers and 30% by mass of the polyester fibers as potentially non-crimpable fibers were uniformly mixed, and then a semi-random carded web having a basis weight of 52 g/m$^2$ was produced by a conventional method. The steam treatment in Example 1 was performed without performing water stream injecting treatment, whereby a crimped-fiber nonwoven fabric having a basis weight of 131 g/m$^2$ was obtained.
Next, the fibers were composited by thermocompression bonding in the same manner as in Example 1, except that: the obtained crimped-fiber nonwoven fabric was rolled out to be used as a support similarly to the support used for the second fiber layer in Example 1; and was laminated by directly spinning on the crimped fiber nonwoven fabric at a basis weight of 10 g/m$^2$, to obtain a fiber sheet having a basis weight of 141 g/m$^2$.

[Comparative Example 1]

Using 100 parts by mass of the styrene-based elastomer resin ("EARNESTON (registered trademark)" CJ101 manufactured by KURARAY PLASTICS CO., Ltd.), melt-blown spinning was performed using general melt-blowing manufacturing equipment, with 400 spinning holes (arranged in a single row) at a spinning temperature of 300°C, an air pressure of 4 MPa, a hole diameter $\varphi$ of 0.3 mm and a single hole discharge rate of 0.4 g/hole/min. The fibers were collected at a speed of 3.2 m/min by using a rotating net conveyor as a support, whereby an elastomer-fiber nonwoven fabric (basis weight: 120 g/m$^2$) was produced. The elastomer-fiber nonwoven fabric was evaluated alone.

[Comparative Example 2]

The crimped-fiber nonwoven fabric in Example 1 was evaluated alone.

[Comparative Example 3]

A fiber sheet was obtained in the same manner as in Example 1, except that the following polypropylene-fiber nonwoven fabric was used as the second fiber layer. The second fiber layer was a polypropylene-fiber nonwoven fabric, which was produced by using 100 parts by mass of a polypropylene resin (MFR (230°C, 2.16 kg)=1100 g/10 minutes), by performing melt-blown spinning using general melt-blowing manufacturing equipment, with 400 spinning holes (arranged in a single row), at a spinning temperature of 285°C, an air temperature of 275°C, and an air pressure of 0.4 MPa, a hole diameter $\varphi$ of 0.3 mm and a single hole discharge rate of 0.2 g/hole/min, and by collecting the fibers at a speed of 12 m/min by using a rotating net conveyor as a support, whereby a polypropylene-fiber nonwoven fabric having a basis weight of 60 g/m$^2$ was obtained.

[Comparative Example 4]

A fiber sheet was obtained in the same manner as in Example 1, except that a nonwoven fabric as the first fiber layer was produced as follows.

(Preparation of First Fiber Layer)

45% by mass of the rayon and 55% by mass of the polyester fibers were uniformly mixed, and then a semi-random carded web having a basis weight of 90 g/m² was produced by a conventional method. This carded web was placed on a punched drum support having an open area ratio of 25% and a hole diameter of 0.3 mm. Then, the carded web was continuously transferred in the longitudinal direction at a speed of 50 m/min while high-pressure water streams were injected from the above so as to perform the entanglement treatment. In this entanglement treatment, two nozzles were used (the distance between the adjacent nozzles: 20 cm), in which orifices each having a hole diameter of 0.10 mm were provided at an interval of 0.6 mm along the width direction of the web. High-pressure water streams were injected from the nozzle in the first row at a water pressure of 3.0 MPa; and high-pressure water streams were injected from the nozzle in the second row at a water pressure of 4.0 MPa. Thus, a nonwoven fabric was obtained.

[Comparative Example 5]

A fiber sheet was obtained in the same manner as in Example 1, except that the speed of the net conveyor at the time of production of the second fiber layer was changed to 1.5 m/min and the single hole discharge rate was changed to 0.2 g/hole/min to obtain an elastomer nonwoven fabric having a basis weight of 100 g/m².
The structures and the physical properties of the obtained fiber sheets are indicated in Table 1.

[Table 1-1]

| | | | Unit | Example 1 | Example 2 | Example 3 | Example 4 |
|---|---|---|---|---|---|---|---|
| First fiber layer | Fiber 1 | | | Crimped fiber | Crimped fiber | Crimped fiber | Crimped fiber |
| | Fiber 2 | | | - | - | - | Rayon fiber |
| | Average fiber diameter | | (μm) | 13 | 13 | 11 | 13 |
| | Proportion of crimped fiber | | (% by mass) | 100 | 100 | 100 | 60 |
| | Basis weight | | (g/m²) | 90 | 90 | 90 | 100 |
| | Thickness | | mm | 0.96 | 1.10 | 0.92 | 1.25 |
| Second fiber layer | Resin | | | Styrene-based TPE | Urethane-based TPE | Styrene-based TPE | Styrene-based TPE |
| | Basis weight | | (g/m²) | 100 | 70 | 100 | 100 |
| | Fiber diameter | | (μm) | 18 | 10 | 18 | 18 |
| | Thickness | | mm | 0.32 | 0.27 | 0.32 | 0.32 |
| Fiber sheet | Basis weight | | (g/m²) | 190 | 160 | 190 | 200 |
| | Thickness ratio (second fiber layer)/(first fiber layer) | | | 25/75 | 20/80 | 26/74 | 21/79 |
| | Breaking strength (MD) | | (N/50 mm) | 18.8 | 28.6 | 23.5 | 10.1 |
| | Breaking strength (CD) | | (N/50 mm) | 6.8 | 19.5 | 8.5 | 7.8 |
| | Surface friction coefficient of second fiber layer | | | 7.4 | 5.1 | 7.2 | 7.3 |
| | Recovery rate after 10 cycles of 50% elongation in MD | | (%) | 90.1 | 87.9 | 85.6 | 81.1 |

(continued)

| | | Unit | Example 1 | Example 2 | Example 3 | Example 4 |
|---|---|---|---|---|---|---|
| | Recovery rate after 10 cycles of 100% elongation in CD | (%) | 77.4 | 68.1 | 73.5 | 61.9 |
| | Interlayer peeling strength in MD between first and second fiber layers | (g/cm) | 1.34 | 1.08 | 1.15 | 1.07 |
| | Proportion of joining area | (%) | 14.87 | 14.87 | 14.87 | 14.87 |

[Table 1-2]

| | | Unit | Example 5 | Example 6 | Example 7 | Example 8 |
|---|---|---|---|---|---|---|
| First fiber layer | Fiber 1 | | Crimped fiber | Crimped fiber | Crimped fiber | Crimped fiber |
| | Fiber 2 | | - | - | Rayon fiber | - |
| | Average fiber diameter | ($\mu$m) | 13 | 9 | 13 | 13 |
| | Proportion of crimped fiber | (% by mass) | 100 | 100 | 50 | 100 |
| | Basis weight | (g/m$^2$) | 90 | 90 | 90 | 90 |
| | Thickness | mm | 0.96 | 0.80 | 1.13 | 1.13 |
| Second fiber layer | Resin | | Styrene-based TPE | Styrene-based TPE | Styrene-based TPE | Styrene-based TPE |
| | Basis weight | (g/m$^2$) | 40 | 100 | 100 | 200 |
| | Fiber diameter | ($\mu$m) | 18 | 18 | 18 | 18 |
| | Thickness | mm | 0.13 | 0.32 | 0.32 | 0.80 |
| Fiber sheet | Basis weight | (g/m$^2$) | 130 | 190 | 190 | 290 |
| | Thickness ratio (second fiber layer)/(first fiber layer) | | 12/88 | 29/71 | 22/78 | 41/59 |
| | Breaking strength (MD) | (N/50 mm) | 17.0 | 20.1 | 30.2 | 19.5 |
| | Breaking strength (CD) | (N/50 mm) | 6.6 | 6.4 | 23.2 | 7.1 |
| | Surface friction coefficient of second fiber layer | | 4.8 | 7.3 | 7.4 | 7.4 |
| | Recovery rate after 10 cycles of 50% elongation in MD | (%) | 90.5 | 77.0 | 72.9 | 95.0 |
| | Recovery rate after 10 cycles of 100% elongation in CD | (%) | 50.0 | 66.2 | 55.7 | 81.6 |
| | Interlayer peeling strength in MD between first and second fiber layers | (g/cm) | 0.48 | 1.08 | 0.68 | 0.86 |
| | Proportion of joining area | (%) | 14.87 | 14.87 | 14.87 | 14.87 |

[Table 1-3]

| | | Unit | Example 9 | Example 10 | Comparative Example 1 | Comparative Example 2 |
|---|---|---|---|---|---|---|
| First fiber layer | Fiber 1 | | Crimped fiber | Crimped fiber | - | Crimped fiber |
| | Fiber 2 | | - | PET | - | - |
| | Average fiber diameter | (μm) | 13 | 12 | - | 13 |
| | Proportion of crimped fiber | (% by mass) | 100 | 70 | - | 100 |
| | Basis weight | (g/m²) | 90 | 131 | - | 90 |
| | Thickness | mm | 1.13 | 3.70 | - | 0.92 |
| Second Fiber layer | Resin | | Styrene-based TPE | Styrene-based TPE | Styrene-based TPE | - |
| | Basis weight | (g/m²) | 200 | 10 | 120 | - |
| | Fiber diameter | (μm) | 18 | 18 | 18 | - |
| | Thickness | mm | 1.15 | 0.10 | 0.50 | - |
| Fiber sheet | Basis weight | (g/m²) | 290 | 141 | 120 | 90 |
| | Thickness ratio (second fiber layer)/(first fiber layer) | | 50/50 | 3/97 | - | - |
| | Breaking strength (MD) | (N/50 mm) | 19.0 | 17.0 | 1.6 | 16.9 |
| | Breaking strength (CD) | (N/50 mm) | 6.9 | 6.1 | 1.2 | 4.8 |
| | Surface friction coefficient of second fiber layer | | 6.3 | 4.0 | 7.3 | 3.0 |
| | Recovery rate after 10 cycles of 50% elongation in MD | (%) | 93.0 | 71.3 | 92.2 | 90.2 |
| | Recovery rate after 10 cycles of 100% elongation in CD | (%) | 76.0 | 48.7 | 93.8 | 47.4 |
| | Interlayer peeling strength in MD between first and second fiber layers | (g/cm) | 0.73 | 0.46 | - | - |
| | Proportion of joining area | (%) | 14.87 | 14.87 | - | - |

[Table 1-4]

| | | Unit | Comparative Example 3 | Comparative Example 4 | Comparative Example 5 |
|---|---|---|---|---|---|
| First fiber layer | Fiber 1 | | Crimped fiber | Rayon fiber | Crimped fiber |
| | Fiber 2 | | - | PET | - |
| | Average fiber diameter | (μm) | 13 | 13 | 13 |
| | Proportion of crimped fiber | (% by mass) | 100 | 0 | 100 |
| | Basis weight | (g/m²) | 90 | 90 | 90 |
| | Thickness | mm | 0.83 | 0.55 | 0.96 |

(continued)

|  |  | Unit | Comparative Example 3 | Comparative Example 4 | Comparative Example 5 |
|---|---|---|---|---|---|
| Second fiber layer | Resin | | Polypropylene | Styrene-based TPE | Styrene-based TPE |
| | Basis weight | (g/m²) | 60 | 100 | 100 |
| | Fiber diameter | (μm) | 3 | 18 | 18 |
| | Thickness | mm | 0.50 | 0.32 | 1.08 |
| Fiber sheet | Basis weight | (g/m²) | 160 | 190 | 190 |
| | Thickness ratio (second fiber layer)/(first fiber layer) | | 38/62 | 37/63 | 53/47 |
| | Breaking strength (MD) | (N/50 mm) | 36.0 | 278 | 17.2 |
| | Breaking strength (CD) | (N/50 mm) | 34.5 | 70.0 | 5.3 |
| | Surface friction coefficient of second fiber layer | | 3.5 | 7.4 | 4.3 |
| | Recovery rate after 10 cycles of 50% elongation in MD | (%) | N/A Broken | N/A Broken | N/A Broken |
| | Recovery rate after 10 cycles of 100% elongation in CD | (%) | N/A Broken | N/A Broken | N/A Broken |
| | Interlayer peeling strength in MD between first and second fiber layers | (g/cm) | 2.68 | 2.03 | 0.36 |
| | Proportion of joining area | (%) | 14.87 | 14.87 | 14.87 |

As shown in Table 1, in Comparative Example 1, since the styrene-based elastomer nonwoven fabric was used alone, the surface friction coefficient was high, but the breaking strengths (tensile breaking stresses) were insufficient compared to Examples 1 to 7.

In Comparative Example 2, since the crimped-fiber nonwoven fabric was used alone, the surface friction coefficient could not be increased, and the slip resistance was inferior.

In Comparative Example 3, since the polypropylene nonwoven fabric which is a non-elastomer nonwoven fabric was used as a second fiber layer, the surface friction coefficient could not be increased. Furthermore, the fiber sheet was broken when being stretched, even though the crimped-fiber nonwoven fabric was used as the first fiber layer.

In Comparative Example 4, since the non-crimped-fiber nonwoven fabric was used as the first fiber layer, the fiber sheet was broken when being stretched, even though the styrene-based elastomer nonwoven fabric was used as the second fiber layer.

In Comparative Example 5, since the thickness of the second fiber layer was larger than the thickness of the first fiber layer, the fiber sheet was broken when being stretched, even though the crimped-fiber nonwoven fabric was used as the first fiber layer and the styrene-based elastomer nonwoven fabric was used as the second fiber layer.

Meanwhile, in each of Examples 1 to 9, the breaking strength in each of the MD and the CD was sufficient. Furthermore, even in a case where the first fiber layer repeatedly expanded and contracted in the longitudinal direction of the fiber sheet, the second fiber layer was able to follow the repeated expansion and contraction of the first fiber layer without peeling off, so that the fiber sheet as a whole was able to exhibit repetitive elasticity. Besides, the fiber sheet also exhibited slip resistance since the surface friction coefficient of the fiber sheet had a large value.

Moreover, in each of the Examples, both repetitive elasticity and breaking strength of the fiber sheet in the CD can be improved as compared to Comparative Examples 1 to 5.

INDUSTRIAL APPLICABILITY

As described above, the fiber sheet according to the present invention can be used for various applications such as household items, sporting goods, clothing items, medical, cosmetic, and hygiene materials, and the like.

Although the present invention has been described above in connection with the preferred embodiments thereof with reference to the accompanying drawings, numerous additions, changes, and deletions can be made without departing from the gist of the present invention. Therefore, such additions, changes, and deletions are also construed as included within the scope of the present invention.

[Reference Numerals]

10 ···· fiber sheet
1 ···· first fiber layer
2 ···· second fiber layer

**Claims**

1. A fiber sheet comprising at least:

   a first fiber layer containing crimped fibers each having a coil shape, the first fiber layer having repetitive elasticity in a longitudinal direction of the fiber sheet; and
   a second fiber layer containing elastomer fibers,
   wherein the second fiber layer is integrated with the first fiber layer by fusion or adhesion, and
   a thickness ratio of the second fiber layer to the first fiber layer, expressed as (second fiber layer) / (first fiber layer), is from 3/97 to 50/50.

2. The fiber sheet according to claim 1, wherein the second fiber layer has a basis weight of 10 $g/m^2$ or higher.

3. The fiber sheet according to claim 1 or 2, wherein the crimped fibers are derived from ester-based composite fibers.

4. The fiber sheet according to any one of claims 1 to 3, wherein the second fiber layer has a fused intersection between the elastomer fibers.

5. The fiber sheet according to any one of claims 1 to 4, wherein the elastomer fibers are styrene-based elastomer fibers.

6. The fiber sheet according to any one of claims 1 to 5, wherein a proportion of a bonding area between the second fiber layer and the first fiber layer is from 5 to 25% with respect to an area of an entire surface of the second fiber layer.

7. The fiber sheet according to any one of claims 1 to 6, wherein an interlayer peeling strength between the second fiber layer and the first fiber layer is 0.1 g/cm or higher.

8. The fiber sheet according to any one of claims 1 to 7, wherein each of the first fiber layer and the second fiber layer is a nonwoven fabric.

9. The fiber sheet according to claim 8, wherein the first fiber layer is a hydroentangled nonwoven fabric, and the second fiber layer is a melt-blown nonwoven fabric.

10. The fiber sheet according to any one of claims 1 to 9, comprising one or more first fiber layers and one or more second fiber layers,
    wherein a second fiber layer is exposed on at least a part of at least one outermost surface of the fiber sheet.

11. The fiber sheet according to any one of claims 1 to 10, wherein the fiber sheet is a bandage.

Fig. 1

10

1

2

Fig. 2

Fig. 3

1

2

Fig. 4

Fig. 5

Fig. 6

1 PITCH

## INTERNATIONAL SEARCH REPORT

| | International application No. |
|---|---|
| | **PCT/JP2023/006412** |

**A. CLASSIFICATION OF SUBJECT MATTER**

**B32B 5/26**(2006.01)i; **A61F 13/00**(2006.01)i; **B32B 5/02**(2006.01)i; **D04H 1/4374**(2012.01)i
FI: B32B5/26; A61F13/00 355F; B32B5/02 C; D04H1/4374

According to International Patent Classification (IPC) or to both national classification and IPC

**B. FIELDS SEARCHED**

Minimum documentation searched (classification system followed by classification symbols)

B32B5/26; A61F13/00; B32B5/02; D04H1/4374

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2002-1855 A (MARUE NISSAN CO., LTD.) 08 January 2002 (2002-01-08)<br>claims, paragraphs [0001], [0009], [0019], [0026]-[0029], [0038], [0050] | 1-11 |
| A | JP 2009-183363 A (KURARAY KURAFLEX CO., LTD.) 20 August 2009 (2009-08-20)<br>entire text | 1-11 |
| A | JP 2009-97133 A (KURARAY KURAFLEX CO., LTD.) 07 May 2009 (2009-05-07)<br>entire text | 1-11 |
| A | WO 2008/015972 A1 (KURARAY KURAFLEX CO., LTD.) 07 February 2008 (2008-02-07)<br>entire text | 1-11 |

☐ Further documents are listed in the continuation of Box C.     ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 May 2023** | **23 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/006412**

| Patent document cited in search report | | | Publication date (day/month/year) | Patent family member(s) | | | Publication date (day/month/year) |
|---|---|---|---|---|---|---|---|
| JP | 2002-1855 | A | 08 January 2002 | (Family: none) | | | |
| JP | 2009-183363 | A | 20 August 2009 | (Family: none) | | | |
| JP | 2009-97133 | A | 07 May 2009 | (Family: none) | | | |
| WO | 2008/015972 | A1 | 07 February 2008 | US | 2010/0035500 | A1 | |
| | | | | whole document | | | |
| | | | | EP | 2058424 | A1 | |
| | | | | KR | 10-2009-0048457 | A | |
| | | | | CN | 101522972 | A | |
| | | | | AU | 2007279816 | A | |
| | | | | TW | 200824655 | A | |
| | | | | ES | 2446246 | T | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022029399 A

- JP 6560683 B

**Non-patent literature cited in the description**

- Test methods for man-made staple fibres. *JIS L 1015*
- Test methods for general nonwovens. *JIS L 1913*

- Plastics - Film and sheeting - Determination of the coefficients of friction. *JIS K 7125*, 1999